# EUROPÄISCHE PATENTSCHRIFT

(11) **EP 1 441 851 B1**
(45) Veröffentlichungstag und Bekanntmachung des Hinweises auf die Patenterteilung: **31.08.2005**
(21) Anmeldenummer: 02779497.3
(22) Anmeldetag: 18.10.2002
(51) Int. Cl.: B01J 23/745, C07C 209/48

(54) **VERFAHREN ZUR KATALYTISCHEN HYDRIERUNG EINER NITRIL-GRUPPE UNTER VERWENDUNG EINER EISEN-ENTHALTENDEN MASSE**
PROCESS FOR CATALYTICALLY HYDROGENATING NITRILES USING AN IRON-CONTAINING MATERIAL
PROCEDE D'HYDROGENATION CATALYTIQUE DE NITRILES EN PRESENCE D'UNE MASSE CONTENANT DU FER

(30) Priorität: 23.10.2001 DE 10151559
(43) Veröffentlichungstag der Anmeldung: 04.08.2004
(73) Patentinhaber: BASF Aktiengesellschaft, 67056 Ludwigshafen (DE)
(72) Erfinder: ANSMANN, Andreas, 69168 Wiesloch (DE); BENISCH, Christoph, 68165 Mannheim (DE); BASSLER, Peter, 68519 Viernheim (DE); FISCHER, Rolf-Hartmuth, 69121 Heidelberg (DE); MAIXNER, Stefan, 68723 Schwetzingen (DE); MELDER, Johann-Peter, 67459 Böhl-Iggelheim (DE); LUYKEN, Hermann, 67069 Ludwigshafen (DE)
(86) Internationale Anmeldenummer: PCT/EP2002/011669
(87) Internationale Veröffentlichungsnummer: WO 2003/035250

(56) Entgegenhaltungen:
- EP-A- 0 391 743
- WO-A-98/11059
- WO-A-99/44984
- DE-A- 2 429 293
- US-A- 4 714 641
- US-A- 5 846 507

## Beschreibung

Die vorliegende Erfindung betrifft eine als Katalysator geeignete Masse enthaltend
a) Eisen oder eine Mischung enthaltend Eisen und eine Verbindung auf der Basis von Eisen
   wobei das Eisen eine durchschnittliche mittlere Kristallitgröße im Bereich von 1 bis 35 nm, gemessen mittels Röntgenbeugung, aufweist.
   Ferner betrifft sie ein Verfahren zur Herstellung einer solchen Masse, sowie ein Verfahren zur Hydrierung einer Nitril-Gruppe in einer eine Nitril-Gruppe enthaltenden Verbindung zu einer Amino-Gruppe in Gegenwart einer solchen Masse als Katalysator.
   Es ist allgemein bekannt, beispielsweise aus Weissermel/Arpe, Industrielle Organische Chemie, Verlag Chemie, 3. Auflage, 1988, Seite 266, oder WO-A-96/20166, daß Adipodinitril in Gegenwart eines überwiegend Eisen enthaltenden Katalysators zu einem Gemisch aus 6-Aminocapronitril und Hexamethylendiamin oder nur Hexamethylendiamin alleine hydriert werden kann.
   6-Aminocapronitril und Hexamethylendiamin stellen wichtige Vorstufen für die Herstellung technisch bedeutsamer Polymere dar, wie Nylon 6 oder Nylon 6.6.
   Wünschenswert für den Katalysator ist eine hohe mechanische Festigkeit, eine lange Katalysatorstandzeit, eine hohe Raum-Zeit-Ausbeute an den Wertprodukten 6-Aminocapronitril und Hexamethylendiamin oder Hexamethylendiamin allein und insbesondere ein möglichst geringer Gehalt an unerwünschten Nebenprodukten bei der genannten Hydrierung.
   Solche unerwünschten Nebenprodukte lassen sich nur mit hohem Aufwand von dem Wertprodukt abtrennen.
   So bilden sich beispielsweise im Falle der Hydrierung von Adipodinitril zu 6-Aminocapronitril und Hexamethylendiamin in wechselnden Mengen unter anderem Tetrahydroazepin (THA), 1-Amino-2-cyanocyclopenten (ICCP), 2-Aminomethylcyclopentylamin (AMCPA), 1,2-Diaminocyclohexan (DCH) und Bishexamethylentriamin (BHMTA). Aus US-A 3 696 153 ist bekannt, dass sich AMCPA und DCH nur sehr schwer von Hexamethylendiamin abtrennen lassen. Zudem führen vor allem große Mengen an AMCPA, DCH und THA zu einem hohen Destillationsaufwand, der sich in beträchtlichen Investitions- und Energiekosten niederschlägt.
   WO 98/11059 offenbart ein Verfahren zur Herstellung von alpha, omega-Aminonitrilen durch Hydrierung der entsprechenden Nitrile an eisenhaltigen Katalysatoren, das neben hohen Katalysatorstandzeiten und Selektivitäten auch geringe Nebenproduktgehalte ermöglicht. So wird im Beispiel 2 bei einer Temperatur von 80°C eine Gesamtselektivität von 98,9% Hexamethylendiamin und 6-Aminocapronitril bei Gehalten an DCH von 3700 ppm, AMCPA von 430 ppm und ICCP von 80 ppm bezogen auf HMD erzielt. Nachteilig an dem offenbarten Verfahren ist der geringe Umsatz an Adipodinitril, der bei 80°C nur 47,3% beträgt. Somit ist die Raum-Zeit-Ausbeute dieses Verfahrens nur unbefriedigend.
   Aufgabe der vorliegenden Erfindung war es daher, als Katalysator geeignete, Eisen enthaltende Massen bereitzustellen, in deren Gegenwart die Hydrierung von Nitrilen zu Aminen auf technisch einfache Weise unter Vermeidung der genannten Nachteile ermöglicht wird.
   Demgemäß wurden die eingangs definierten Massen, Verfahren zu ihrer Herstellung und Verfahren zur Hydrierung einer Nitril-Gruppe in einer eine Nitril-Gruppe enthaltenden Verbindung zu einer Amino-Gruppe in Gegenwart einer solchen Masse als Katalysator gefunden.
   Erfindungsgemäß weist das Eisen in den Massen eine durchschnittliche mittlere Kristallitgröße von mindestens 1, vorzugsweise mindestens 5, insbesondere mindestens 10 nm auf.
   Erfindungsgemäß weist das Eisen in den Massen eine durchschnittliche mittlere Kristallitgröße von höchstens 35, vorzugsweise höchstens 30 nm, auf.
   Im Sinne der vorliegenden Erfindung gilt die durchschnittliche mittlere Kristallitgröße so, wie sie durch Röntgenbeugung am Diffraktometer D5000 Theta/Theta (Firma Siemens, Deutschland), Auswertesoftware TOPAS, bestimmt ist.
   In einer weiteren bevorzugten Ausführungsform kann die oxidische Masse zusätzlich c) von 0 bis 0,5 Gew.-%, vorzugsweise 0,05 bis 0,4 Gew.-%, insbesondere 0,1 bis 0,2 Gew.-%, bezogen auf a), einer Verbindung auf der Basis eines Alkali- oder Erdalkalimetalls, vorzugsweise ausgewählt aus der Gruppe bestehend aus Lithium, Natrium, Kalium, Rubidium, Cäsium, Magnesium und Calcium oder deren Gemische, enthalten.
   In einer weiteren bevorzugten Ausführungsform kann die oxidische Masse zusätzlich b) von 0,01 bis 5 Gew.-%, vorzugsweise 0,5 bis 4 Gew.-%, insbesondere 1 bis 3 Gew.-%, bezogen auf a), eines Promotors auf der Basis von 1, 2, 3, 4, oder 5 Elementen ausgewählt aus der Gruppe bestehend aus Aluminium, Silizium, Zirkonium, Titan und Vanadium oder deren Gemische, insbesondere eines Promotors auf der Basis von 1, 2 oder 3 Elementen ausgewählt aus der Gruppe bestehend aus Aluminium, Silizium und Titan oder deren Gemische, und
c) von 0 bis 0,5 Gew.-%, vorzugsweise 0,05 bis 0,4 Gew.-%, insbesondere 0,1 bis 0,2 Gew.-%, bezogen auf a), einer Verbindung auf der Basis eines Alkali- oder Erdalkalimetalls, vorzugsweise ausgewählt aus der Gruppe bestehend aus Lithium, Natrium, Kalium, Rubidium, Cäsium, Magnesium und Calcium, enthalten.

In einer weiteren bevorzugten Ausführungsform kann die oxidische Masse zusätzlich d) 0,001 bis 1 Gew.-%, vorzugsweise 0,001 bis 3 Gew.%, insbesondere 0,01 bis 0,2 Gew.-%, bezogen auf a), Mangan enthalten.

Bei den erfindungsgemäßen Katalysatoren kann es sich um Voll- oder Trägerkatalysatoren handeln. Als Trägermaterial kommen beispielsweise Oxide, wie Aluminiumoxid, Lanthanoxid, Titandioxid, Zirkondioxid, Magnesiumoxid, Zinkoxid, und Zeolithe sowie Aktivkohle oder Mischungen davon in Betracht.

Die erfindungsgemäßen Massen sind vorteilhaft dadurch erhältlich, daß man einen Vorläufer, der Eisen, Sauerstoff und gegebenenfalls eine oder mehrere der Anteile b), c) und d) enthält, wobei der Vorläufer ein atomares Verhältnis von zweiwertigem Eisen zu dreiwertigem Eisen von höchstens 0,5 aufweist, in Gegenwart eines Reduktionsmittels bis mindestens zum Schmelzpunkt des Vorläufers erhitzt,abkühlt auf 25°C unter Erhalt einer Masse mit einem Verhältnis von zweiwertigem Eisen zu dreiwertigem Eisen im Bereich von mehr als 0,5 bis 5,5, vorzugsweise im Bereich von 0,57 bis 2,2, und dann die dabei erhaltene Masse mit einem Wasserstoff enthaltenden Gas bei einer Temperatur im Bereich von 200 bis 500°C reduziert.

Der Gehalt der Vorstufe der erfindungsgemäßen Masse an zweiwertigem Eisen und dreiwertigem Eisen ist im Sinne der vorliegenden Erfindung bestimmt durch Ermittlung der Anteile der vorliegenden kristallinen Phasen durch Röntgenbeugung am Diffraktometer D5000 Theta/Theta (Firma Siemens, Deutschland), Auswertesoftware TOPAS.

In einer bevorzugten Ausführungsform enthält die Vorstufe der erfindungsgemäßen Masse zweiwertiges und dreiwertiges Eisen in Form von Magnetit, der im Idealfall mit der Formel Fe₃O₄, also als Fe(II)Fe(III)₂O₄, beschrieben werden kann, und das darüber hinausgehende zweiwertige Eisen teilweise oder vollständig in Form von Wüstit. In einer besonders bevorzugten Ausführungsform liegen mehr als 0 bis zu 90 %, insbesondere 1,2 bis 77 %, des zweiwertigen Eisens in Form von Wüstit vor. Unter Wüstit wird im Sinne der vorliegenden Erfindung ein Eisenoxid der Formel Fe₁₋ₓO mit 0 = x = 0,16 verstanden.

Der Gehalt an Wüstit ist bestimmt im Sinne der vorliegenden Erfindung durch Ermittlung der Anteile der vorliegenden kristallinen Phasen durch Röntgenbeugung am Diffraktometer D5000 Theta/Theta (Firma Siemens, Deutschland), Auswertesoftware TOPAS.

Vorteilhaft kann man die Vorstufe der erfindungsgemäßen Masse erhalten, indem man einen Vorläufer, der Eisen, Sauerstoff und gegebenenfalls eine oder mehrere der Anteile b), c) und d) enthält, wobei der Vorläufer ein geringeres atomares Verhältnis von zweiwertigem Eisen zu dreiwertigem Eisen aufweist als in a) definiert, in Gegenwart eines Reduktionsmittels bis mindestens zum Schmelzpunkt des Vorläufers erhitzt.

Als Eisen und Sauerstoff enthaltende Vorläufer können Eisenoxid, Eisenhydroxid oder Eisenoxyhxdroxid enthaltende Vorläufer, wie Eisen-(III)-oxid, Eisen (II,III)-oxid, Eisen-(II)-oxid, Eisen-(II)-hydroxid, Eisen-(III)-hydroxid oder Eisenoxidhydroxid wie FeOOH eingesetzt werden. Verwendet werden können synthetisch hergestellte oder natürliche Eisenoxide, Eisenhydroxide oder Eisenoxyhydroxide, wie Magneteisenstein (Magnetit), der im Idealfall als Fe₃O₄ beschrieben werden kann, Brauneisenstein, der im Idealfall als Fe₂O₃*H₂O beschrieben werden kann, oder Roteisenstein (Hämatit), der im Idealfall als Fe₂O₃ beschrieben werden kann.

Die gegebenenfalls vorhandenen einen oder mehreren Anteile an b), c) und d) können in dem Vorläufer in Form der Oxide, Hydroxide, anderer Salze anorganischer Säuren, wie Nitrate, Chloride, Carbonate, Sulfate, oder Salze organischer Säuren, wie Formiate, Acetate, vorliegen.

In einer bevorzugten Ausführungsform kommt als Vorläufer ein natürliches Magnetiterz in Betracht. Die Zusammensetzung eines solchen Magnetiterzes kann durch die Zugabe von einem oder mehreren Anteilen an c), d) und e) gewünschtenfalls modifiziert werden.

In einer weiteren bevorzugten Ausführungsform kommt ein Reduktionsmittel ausgewählt aus der Gruppe bestehend aus Eisen, einem oder mehrerer der in b) genannten Elemente, einem oder mehreren der in c) genannten Elemente, Mangan, Kohlenstoff oder deren Gemische, vorzugsweise ausgewählt aus der Gruppe bestehend aus Eisen, Aluminium, Magnesium, Kohlenstoff oder deren Gemische, insbesondere Eisen in Betracht.

Die zur Einstellung des erfindungsgemäßen Verhältnisses von zweiwertigem Eisen zu dreiwertigem Eisen in der oxidischen Masse erforderliche Menge an Reduktionsmittel kann dabei in wenigen einfachen Vorversuchen leicht ermittelt werden.

Verwendet man Eisen als Reduktionsmittel, so hat sich der Zusatz von 1 bis 50 Gew.-% Eisen, bezogen auf die im Vorläufer in Summe vorliegende Eisen- und Sauerstoffmenge, als besonders vorteilhaft erwiesen.

Aus der Vorstufe der erfindungsgemäßen Masse können Träger- oder Vollkatalysatoren, vorzugsweise Vollkatalysatoren, wie sie vorzugsweise für die Hydrierung, insbesondere für die Hydrierung von Nitril-Gruppen zu Amin-Gruppen, beispielsweise von Adipodinitril teilweise oder vollständig zu 6-Aminocapronitril, wie einem Gemisch aus 6-Aminocapronitril und Hexamethylendiamin, oder teilweise oder vollständig zu Hexamethylendiamin, eingesetzt werden können, durch Behandlung in einer reduzierenden Atmosphäre erhalten werden ("Aktivierung"), beispielsweise indem man sie bei einer Temperatur im Bereich von 200 bis 500°C, vorzugsweise 250 bis 450°C, 2 bis 120 Stunden einer Wasserstoff-Atmosphäre oder einer Gasmischung, enthaltend Wasserstoff und ein Inertgas, wie Stickstoff, aussetzt. Die Katalysatorbelastung sollte hierbei bevorzugt 2000 bis 10000 N1 pro 1 Katalysator pro Stunde betragen.

Vorteilhaft kann man die Aktivierung direkt im Synthesereaktor durchführen, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 65°C, mittels Sauerstoff-Stickstoff-Mischungen, wie Luft, wegfällt. Die Aktivierung passivierter Katalysatoren nimmt man bevorzugt im Synthesereaktor bei einer Temperatur im Bereich von 180 bis 500°C, vorzugsweise 200 bis 350°C, in einer Wasserstoff-haltigen Atmosphäre vor.

Die Katalysatoren können als Festbettkatalysatoren in Sumpf- oder Rieselfahrweise oder als Suspensionskatalysatoren eingesetzt werden.

Als Ausgangsstoffe für die Hydrierung können vorteilhaft aliphatische alpha,omega-Dinitrile der allgemeinen Formel

NC-(CH₂)ₙ-CN

mit n eine ganze Zahl von 1 bis 10, insbesondere 2, 3, 4, 5 und 6 bedeutet, eingesetzt werden. Besonders bevorzugte Verbindungen sind Bernsteinsäuredinitril, Glutarsäuredinitril, Adipinsäuredinitril ("Adipodinitril"), Pimelinsäuredinitril und Korksäuredinitril ("Suberonitril"), ganz besonders bevorzugt Adipodinitril. Vorteilhaft können die vorstehend beschriebenen Dinitrile vorzugsweise in Gegenwart eines flüssigen Verdünnungsmittels unter Verwendung eines der genannten Katalysatoren partiell zu alpha,omega-Aminonitrilen der allgemeinen Formel

NC-(CH₂)ₙ-CH₂-NH₂

hydriert werden, wobei n die vorstehend genannte Bedeutung hat.

Besonders bevorzugte Aminonitrile sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Führt man die Umsetzung in einer Suspension durch, so wählt man üblicherweise Temperaturen im Bereich von 40 bis 150°C, vorzugsweise von 50 bis 100°C, besonders bevorzugt von 60 bis 90°C; den Druck wählt man im allgemeinen im Bereich von 2 bis 30, vorzugsweise von 3 bis 30, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275 min, vorzugsweise von 70 bis 200 min.

Bei der Suspensionsfahrweise setzt man als flüssiges Verdünnungsmittel bevorzugt Ammoniak, Amine, Diamine und Triamine mit 1 bis 6 C-Atomen in einer Aminseitenkette wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise 30 bis 80, besonders bevorzugt von 40 bis 70 Gew.-%, bezogen auf die Summe von Dinitril und flüssigem Verdünnungsmittel.

Die Menge an Katalysator wählt man im allgemeinen so, daß die Katalysator-Menge im Bereich von 1 bis 50, bevorzugt 5 bis 20 Gew.-%, bezogen auf die eingesetzte Menge an Dinitril, beträgt.

Die Suspensionshydrierung kann man diskontinuierlich oder bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150°C, vorzugsweise von 30 bis 90°C und einen Druck in der Regel im Bereich von 2 bis 40, vorzugsweise von 3 bis 30 MPa wählt. Bevorzugt führt man die partielle Hydrierung in Gegenwart eines flüssigen Verdünnungsmittels, bevorzugt Ammoniak, Amine, Diamine und Triamine mit 1 bis 6 C-Atomen in einer Aminseitenkette wie Trimethylamin, Triethylamin, Tripropylamin und Tributylamin oder Alkohole, insbesondere Methanol und Ethanol, besonders bevorzugt Ammoniak durch. In einer bevorzugten Ausführungsform wählt man einen Gehalt an Ammoniak im Bereich von 1 bis 10, vorzugsweise von 2 bis 6 g pro g Dinitril. Bevorzugt wählt man dabei eine Katalysatorbelastung im Bereich von 0,1 bis 2,0, vorzugsweise 0,3 bis 1,0 kg Dinitril pro Liter Katalysator pro Stunde. Auch hier kann man durch Veränderung der Verweilzeit den Umsatz und damit die Selektivität einstellen.

Bei der partiellen Hydrierung erhält man eine Mischung, die Dinitril, alpha,omega-Aminonitril und alpha,omega-Diamin enthält. Bei der vollständigen Hydrierung enthält man eine Mischung, die alpha,omega-Diamin enthält.

Die Abtrennung des Wertproduktes oder der Wertprodukte aus diesen Mischungen kann in an sich für solche Mischungen bekannter Weise, beispielsweise durch Destillation oder Extraktion, erfolgen. Solche Trennverfahren sind beispielsweise in WO 96/20166, WO 98/11059, WO 99/44983 und WO 99/44984 beschrieben.

### Beispiele

### Beispiel 1

Herstellung einer erfindungsgemäßen oxidischen Masse

Die Masse wurde hergestellt durch Aufschmelzen einer Mischung von 900 kg eines Magnetiterzes und 75 kg Eisenpulver bei 1550°C an Luft. Nach Abkühlen der Schmelze auf Raumtemperatur und Erstarren wies die Masse folgende Zusammensetzung auf:
72 Gew.-% Eisen, 0,17 Gew.-% Mangan, 0,08 Gew.-% Aluminium, 0,03 Gew.-% Calcium, 0,05 Gew.-% Magnesium, 0,12 Gew.-% Silizium, 0,01 Gew.-% Titan, Rest Sauerstoff. Der Wüstitgehalt betrug 44 % bezogen auf Gesamtgewicht. Das atomare Verhältnis von zweiwertigem Eisen zu dreiwertigem Eisen betrug 1,76.

### Beispiel 2

### Herstellung eines Katalysators

Die Masse gemäß Beispiel 1 wurde in einem Backenbrecher zerkleinert. Eine Siebfraktion von 1,5 bis 3 mm wurde ausgesiebt und im Wasserstoff/Stickstoff-Strom innerhalb von 72 Stunden bei 450°C reduziert. Nach Abkühlen unter Stickstoff wurde der Katalysator mit einem Gemisch aus 1 % Luft in Stickstoff innerhalb von 24 Stunden passiviert, wobei die Temperatur innerhalb der Katalysatorschüttung bei dieser exothermen Reaktion unter 65°C gehalten wurde.

Die durchschnittliche mittlere Kristallitgröße des Eisens betrug 29,5 nm.

### Beispiel 3

### Hydrierung von Adipodinitril im Festbett

Drei in Reihe geschaltete Rohrreaktoren (Gesamtlänge 4,5 m, d = 6 mm) wurden mit 141 ml (239 g) des nach Beispiel 2 hergestellten Katalysators (1,5-3 mm Splitt) befüllt und anschließend drucklos im Wasserstoffstrom (200 l/h) reduziert. Hierzu wurde die Temperatur innerhalb von 24 h von 70°C auf 340°C angehoben und anschließend 72 h bei 340°C gehalten. Nach Absenken der Temperatur wurde dem Reaktor bei 250 bar ein Gemisch aus 75,0 ml/h ADN, 370 ml/h NH₃ und 200 Nl/h H₂ zugeführt.

Unter den genannten Bedingungen wurden in Abhängigkeit von der Temperatur folgende Ergebnisse erhalten:

| Temp. [°C] | ADN-Umsatz [%] | ACN-Selektivität [%] | HMD-Selektivität [%] | ACN- + HMD-Selektivität [%] |
|---|---|---|---|---|
| | | | | |
| 80 | 57,9 | 79,58 | 19,37 | 99,0 |
| 90 | 73,0 | 71,0 | 28,0 | 99,0 |
| 107 | 100,0 | 0,9 | 98,9 | 99,8 |

### Vergleichsbeispiel 1

### Katalysatorherstellung

Es wurde ein Katalysator gemäß WO 98/11059, Beispiel 2a hergestellt.

Die durchschnittliche mittlere Kristallitgröße des Eisens betrug 37 nm.

### Vergleichsbeispiel 2

### Hydrierung von Adipodinitril im Festbett

Mit dem in Vergleichsbeispiel 1 hergestellten Katalysator wurde Adipodinitril im Festbett gemäß WO 98/11059, Beispiel 2b hydriert.

### Es wurden folgende Ergebnisse erhalten:

| Temp. [°C] | ADN-Umsatz [%] | ACN-Selektivität [%] | HMD-Selektivität [%] | ACN- + HMD-Selektivität [%] |
|---|---|---|---|---|
| | | | | |
| 80 | 47,3 | 80,48 | 18,57 | 98,9 |
| 90 | 72,1 | 67,3 | 31,7 | 99,0 |
| 107 | 99,9 | 0,6 | 98,6 | 99,2 |

## Patentansprüche

1. Verfahren zur Hydrierung einer Nitril-Gruppe in einer eine Nitril-Gruppe enthaltenden Verbindung zu einer Amino-Gruppe in Gegenwart eines Katalysators, **dadurch gekennzeichnet, daß** man als Katalysator eine Masse einsetzt, enthaltend
a) Eisen oder eine Mischung enthaltend Eisen und eine Verbindung auf der Basis von Eisen
wobei das Eisen eine durchschnittliche mittlere Kristallitgröße im Bereich von 1 bis 35 nm, gemessen mittels Röntgenbeugung, aufweist.

2. Verfahren nach Anspruch 1, wobei die Masse zusätzlich
b) von 0,01 bis 5 Gew.-%, bezogen auf a), eines Promotors auf der Basis von 1, 2, 3, 4, oder 5 Elementen ausgewählt aus der Gruppe bestehend aus Aluminium, Silizium, zirkonium, Titan und Vanadium und
c) von 0 bis 0,5 Gew.-%, bezogen auf a), einer Verbindung auf der Basis eines Alkali- oder Erdalkalimetalls
enthält.

3. Verfahren nach Anspruch 1, wobei die Masse zusätzlich
b) von 0,01 bis 5 Gew.-%, bezogen auf a), eines Promotors auf der Basis von 1, 2 oder 3 Elementen ausgewählt aus der Gruppe bestehend aus Aluminium, Silizium und Vanadium und
c) von 0 bis 0,5 Gew.-%, bezogen auf a), einer Verbindung auf der Basis eines Alkali- oder Erdalkalimetalls
enthält.

4. Verfahren nach Anspruch 1, wobei die Masse zusätzlich c) von 0 bis 0,5 Gew.-%, bezogen auf a), einer Verbindung auf der Basis eines Alkali- oder Erdalkalimetalls enthält.

5. Verfahren nach den Ansprüchen 1 bis 4, wobei die Masse zusätzlich
d) von 0,001 bis 1 Gew.-% Mangan in Form einer Verbindung auf der Basis von Mangan
enthält.

6. Verfahren nach den Ansprüchen 1 bis 5, wobei die Masse als Vollkatalysator vorliegt.

7. Verfahren nach den Ansprüchen 1 bis 5, wobei die Masse zusammen mit einem Träger als Trägerkatalysator vorliegt.

8. Verfahren nach den Ansprüchen 1 bis 7, wobei man als Nitril-Gruppe enthaltende Verbindung Adipodinitril einsetzt.

9. Verfahren nach den Ansprüchen 1 bis 8, wobei man Adipodinitril teilweise oder vollständig zu 6-Aminocapronitril hydriert.

10. Verfahren nach den Ansprüchen 1 bis 9, wobei man Adipodinitril teilweise oder vollständig zu Hexamethylendiamin hydriert.

## Claims

1. A process for hydrogenating a nitrile group in a compound containing a nitrile group to form an amino group in the presence of a catalyst, wherein the catalyst used is a composition which comprises
a) iron or a mixture comprising iron and a compound based on iron,
wherein the iron has an average mean crystallite size in the range from 1 to 35 nm, measured by means of X-ray diffraction.

2. A process as claimed in claim 1, wherein the composition further comprises
b) from 0.01 to 5% by weight, based on a), of a promoter based on 1, 2, 3, 4 or 5 elements selected from the group consisting of aluminum, silicon, zirconium, titanium and vanadium and
c) from 0 to 0.5% by weight, based on a), of a compound based on an alkali metal or alkaline earth metal.

3. A process as claimed in claim 1, wherein the composition further comprises
b) from 0.01 to 5% by weight, based on a), of a promoter based on 1, 2 or 3 elements selected from the group consisting of aluminum, silicon and vanadium and
c) from 0 to 0.5% by weight, based on a), of a compound based on an alkali metal or alkaline earth metal.

4. A process as claimed in claim 1, wherein the composition further comprises c) from 0 to 0.5% by weight, based on a), of a compound based on an alkali metal or alkaline earth metal.

5. A process as claimed in any of claims 1 to 4, wherein the composition further comprises d) from 0.001 to 1% by weight of manganese in the form of a compound based on manganese.

6. A process as claimed in any of claims 1 to 5, wherein the composition is in the form of an unsupported catalyst.

7. A process as claimed in any of claims 1 to 5, wherein the composition is combined with a support to form a supported catalyst.

8. A process as claimed in any of claims 1 to 7, wherein the compound containing a nitrile group which is used is adiponitrile.

9. A process as claimed in any of claims 1 to 8, wherein adiponitrile is partly or wholly hydrogenated to 6-aminocapronitrile.

10. A process as claimed in any of claims 1 to 9, wherein adiponitrile is partly or wholly hydrogenated to hexamethylenediamine.

## Revendications

1. Procédé d'hydrogénation d'un groupe nitrile dans un composé contenant un groupe nitrile en un groupe amino, en présence d'un catalyseur, **caractérisé en ce que**, comme catalyseur, on met en oeuvre une masse contenant
a) du fer ou un mélange contenant du fer et un composé à base de fer,
le fer présentant une grosseur de cristallite médiane moyenne de l'ordre de 1 à 35 nm, mesurée au moyen d'une diffraction à rayons X.

2. Procédé suivant la revendication 1, dans lequel la masse contient en supplément
b) de 0,01 à 5% en poids, par rapport à a), d'un promoteur à base de 1, 2, 3, 4 ou 5 éléments choisis parmi le groupe constitué de l'aluminium, du silicium, du zirconium, du titane et du vanadium, et
c) de 0 à 0,5% en poids, par rapport à a), d'un composé à base d'un métal alcalin ou alcalino-terreux.

3. Procédé suivant la revendication 1, dans lequel la masse contient en supplément
b) de 0,01 à 5% en poids, par rapport à a), d'un promoteur à base de 1, 2 ou 3 éléments choisis parmi le groupe constitué de l'aluminium, du silicium et du vanadium, et
c) de 0 à 0,5% en poids, par rapport à a), d'un composé à base d'un métal alcalin ou alcalino-terreux.

4. Procédé suivant la revendication 1, dans lequel la masse contient en supplément
c) de 0 à 0,5% en poids, par rapport à a), d'un composé à base d'un métal alcalin ou alcalino-terreux.

5. Procédé suivant les revendications 1 à 4, dans lequel la masse contient en supplément
d) de 0,001 à 1% en poids de manganèse sous forme d'un composé à base de manganèse.

6. Procédé suivant les revendications 1 à 5, dans lequel la masse se présente sous la forme d'un catalyseur massique.

7. Procédé suivant les revendications 1 à 5, dans lequel la masse se présente, conjointement à un support, sous la forme d'un catalyseur sur support.

8. Procédé suivant les revendications 1 à 7, dans lequel on met en oeuvre de l'adipodinitrile comme composé contenant un groupe nitrile.

9. Procédé suivant les revendications 1 à 8, dans lequel on hydrogène de l'adipodinitrile partiellement ou totalement en 6-aminocapronitrile.

10. Procédé suivant les revendications 1 à 9, dans lequel on hydrogène de l'adipodinitrile partiellement ou totalement en hexaméthylènediamine.
